Europäisches Patentamt

European Patent Office

Office européen des brevets

(11) Publication number: **0 546 587 A1**

# EUROPEAN PATENT APPLICATION

(21) Application number: **92121267.6**

(22) Date of filing: **14.12.92**

(51) Int. Cl.⁵: **A61F 13/00**, D21H 21/56, D21H 23/46

(30) Priority: **13.12.91 US 808265**

(43) Date of publication of application:
**16.06.93 Bulletin 93/24**

(84) Designated Contracting States:
**BE DE GB IE LU**

(71) Applicant: **JOHNSON & JOHNSON INC.**
**2155 Boulevard Pie IX**
**Montreal, Ouebec H1V 2E4(CA)**

(72) Inventor: **Lemay, Martin**
**2640 Langelier, Apt.1**
**Montreal, Ouebec H1N 3A1(CA)**

(74) Representative: **Strehl, Schübel-Hopf,**
**Groening**
**Maximilianstrasse 54 Postfach 22 14 55**
**W-8000 München 22 (DE)**

(54) **Method and apparatus for applying a conditioning agent to a fibrous material and the resulting product thereof.**

(57) Method and apparatus for applying a conditioning agent to a fibrous material to impart a desirable property to the material, such as an enhanced hydrophilicity. The method comprises the steps of depositing wetting agent in a foamed condition on the fibrous material and establishing a pressure differential across the fibrous material to draw the foam of wetting agent therein. By this method a non-uniform dispersion of the wetting agent throughout the fibrous network is obtained, the concentration of wetting agent being highest on the surface of the fibrous material on which the foam is delivered. As a result, the treated fibrous material has a preferential fluid absorption surface. The invention also provides a fluid-absorbent board of peat moss material with main surfaces having different fluid absorption rates.

## FIELD OF THE INVENTION

The invention relates to the art of treating fibrous materials with a conditioning agent to impart desirable properties thereto, such as an enhanced affinity for water, water repellency or others. More specifically, the invention pertains to the field of manufacturing disposable structures for absorbing body exudate, such as sanitary napkins, diapers, adult briefs, urinary pads, tampons, wound dressings and the like, and provides a novel method and apparatus for applying wetting agent (for the purpose of this specification "wetting agent" should be construed to include any substance that enhances the hydrophilicity or wettability of the surface to which it is applied. Similarly, "fluid repelling agent" should be construed to include any substance that enhances the hydrophobicity of the surface to which it is applied) to a fluid absorbent fibrous material, such as peat moss for example. The invention also extends to a fluid absorbent peat moss board with a preferential fluid absorption surface.

## BACKGROUND OF THE INVENTION

The prior art has recognized the potential of peat moss material for use as an absorbent medium in structures for absorbing body exudate. Peat moss material has highly desirable fluid absorption properties such as a remarkable absorption capacity and the ability of "drying" adjacent materials by continuing to pull or wick fluid away from them over a long time period such that virtually all the fluid is collected in the peat moss core. These attributes allow the material to provide highly efficient absorbent components which can be made relatively thin for better fit, comfort and discretion, while being sufficiently absorbent to prevent overflow leakage and garment staining.

The following United States Patents document the use of peat moss material for manufacturing absorbent components for disposable absorbent products:

| PATENT # | INVENTOR | DATE ISSUED |
|---|---|---|
| 4,170,515 | Lalancette et al. | October 9, 1979 |
| 4,215,692 | Levesque | August 5, 1980 |
| 4,226,237 | Levesque | October 7, 1980 |
| 4,305,393 | Nguyen | December 15, 1981 |
| 4,473,440 | Ovans | September 25, 1984 |
| 4,507,122 | Levesque | March 26, 1985 |
| 4,618,496 | Brasseur | October 21, 1986 |
| 4,676,871 | Cadieux et al. | June 30, 1987 |
| 4,992,324 | Dubé | February 12, 1991 |
| 5,053,029 | Yang | October 1, 1991 |

The subject matter of these references is incorporated herein by reference.

Peat moss material can be formed in a highly cohesive board by using any one of the methods disclosed in the above identified prior art. In a board form, the peat moss material is convenient to handle and it can be directly processed in high speed automatic equipment for assembling disposable absorbent products.

More particularly, the method for producing the peat moss board consists of classifying raw peat moss material in particulate form to retain only the particles which are the most absorbent. The screened fraction is sheeted on a Fourdrinier wire in the form of a slurry and de-watered by the application of vacuum. The thus formed board is dried and calendered to increase its density to the desired level. In order to tenderize, soften and improve the flexibility of the calendered peat moss board, it may be subjected to mechanical working such as perf-embossing and micro-corrugating as described in the United States patents 4,559,050 and 4,596,567 to Iskra issued on December 17, 1985 and June 24, 1986 respectively. The disclosure of these patents is incorporated herein by reference.

To increase the fluid absorption rate of the peat moss board in order to enhance its ability to capture on contact a fluid discharge, it is common practise to add a wetting agent to the slurry of peat moss material which increases the hydrophilicity of the peat moss fibres. The wetting agent may be a surfactant which reduces the interfacial tension between the fluid discharge and the fibrous network.

This method for applying the wetting agent is not entirely satisfactory because only a relatively small amount of wetting agent is actually fixed to the peat moss fibers and most of the wetting agent is lost in the dilution fluid of the slurry. A possible solution is to increase the concentration of wetting agent in the slurry, however this is achieved at the expense of rendering the dilution fluid difficult to pump because the wetting agent causes pump impellers to cavitate.

## OBJECTS AND STATEMENT OF THE INVENTION

An object of the present invention is an improved method and apparatus for applying a conditioning agent to a fibrous material for imparting a desirable characteristic to the material.

A more specific object of the invention is a method and apparatus for applying a wetting agent to peat moss material, permitting to fix to the fibrous network a substantial portion of the wetting agent that is being deposited on the peat moss material.

Other objects will become apparent from the ensuing description of the invention.

## SUMMARY OF THE INVENTION

As embodied and broadly described herein, the invention provides a method for applying a conditioning agent to a fibrous material to impart a desirable property thereto, the method comprising the steps of:
- providing a conditioning agent in a foamed condition (for the purpose of this specification, the term "foam" will be used to designate an aggregation of bubbles formed of a liquid substance);
- depositing the conditioning agent on the fibrous material; and
- establishing a pressure differential across the fibrous material to draw the conditioning agent therein.

By this method, a substantial portion of the foam of conditioning agent that is deposited on the fibrous material is permanently absorbed therein. This is due to the nature of the foam mass which looses its cohesiveness as it is drawn within the fibrous network and disperses completely therein. In a dispersed condition, the conditioning agent strongly adheres to the fibers and it is difficult to extract from the fibrous network even at high pressure differential levels. By comparison, when the conditioning agent is incorporated in the dilution fluid of a slurry of the fibrous material to be treated, most of the wetting agent entering the fibrous network is extracted along with the dilution fluid when the slurry is de-moisturized by the application of vacuum, pressure or another agency.

The method in accordance with the invention may be used for treating a large number of different fibrous substrates such as example peat moss, wood pulp fibers, synthetic fibers, textile fibers, paper making fibers and mixtures thereof, with a variety of conditioning agents, such as wetting agents, fluid repelling agents, fire-retarding agents, among others. In all instances, the conditioning agent that is selected should be such as to be capable of being foamed.

As embodied and broadly described herein, the invention provides a method for applying wetting agent to peat moss material, said method comprising the steps of:
- forming an aqueous slurry of peat moss material;
- sheeting said slurry;
- providing wetting agent in a foamed condition;
- depositing said wetting agent on said slurry; and
- establishing a pressure differential across said slurry to de-water said slurry and to draw said wetting agent in said slurry.

For the purpose of this specification, peat moss will be considered as a fibrous material, although in reality it is constituted by small particles which individually may not necessarily have a fibrous identity. However, on a macroscopic scale, the peat moss material has the principal characteristics of a fibrous web and for that reason it will be considered to belong to the general class of fibrous materials.

In a preferred embodiment, the slurry is carried forward on a Fourdrinier wire and partially de-watered to reach a consistency level in the range from about 4 to about 15 % solids. Most preferably, the consistency of the slurry is set at 4.4% solids. The foam of wetting agent is uniformly applied on the moving slurry and it is gradually drawn therein as the slurry passes over additional vacuum slots. For an effective de-watering action and to cause the foam of wetting agent to penetrate deeply in the fibrous network, the suction provided by the vacuum slots is increased progressively along the path of travel of the slurry.

In a preferred embodiment, the wetting agent is foamed by mechanically agitating and aerating an aqueous solution of surfactant.

The density of the foam of wetting agent and the concentration of wetting agent in the foam are selected in accordance with the fibrous material to be treated. For peat moss material, the foam has preferably a density in the range from about 0.015 to about 0.090 grams per cubic centimeter (g/cc) and more preferably a density of 0.023 g/cc.

In a preferred embodiment, the foam density, the concentration of surfactant in the foam and the amount of foam deposited on the slurry are regulated to deliver to the slurry an amount of surfactant which in bone dry state weighs in the range from about 1.4 to about 5.8 grams (g) per kilogram of bone dry solids in the slurry. Most preferably, 2.24 g of surfactant in bone dry state are delivered per kilogram of bone dry solids in the slurry. The foam of wetting agent is applied on the slurry by feeding foam to an open-bottom chamber spaced from the top surface of the slurry by a predetermined distance. The foam delivered in the open-bottom chamber falls on the slurry and is drawn therewith, leaving the open-bottom chamber in the form of a foam layer having a thickness corresponding to the distance separating the open-bottom chamber from the slurry. By varying this distance, the foam application rate is adjusted as desired.

In a preferred embodiment, the top surface of the slurry is heated to disperse residual foam fragments remaining on the slurry that have not penetrated therein. A spray of steam directed at the top surface of the slurry has been found particularly advantageous to achieve this objective.

As embodied and broadly described herein the invention also provides a fluid absorbent board of peat moss material with a preferential fluid absorption surface, the board comprising first and second generally parallel main surfaces, the first main surface having a higher fluid absorption rate than the second main surface and constituting the preferential fluid absorption surface.

It has been observed that the application of wetting agent by the method in accordance with the invention provides a peat moss board where the concentration of wetting agent is highest near the surface on which the foam has been deposited. As a result, the peat moss board has a preferential fluid absorbent surface which is more hydrophilic than the opposite surface of the board and has the ability to take-up fluid more rapidly. This novel absorbent structure may be particularly advantageous for specific applications where the fluid absorption rate of its main surfaces should not be the same.

The peat moss board may also comprise fibrous materials selected from the group consisting of sulfate, sulfite, Kraft wood pulp, mechanical wood pulp, rayon, polyester, nylon, acrylic, and mixtures thereof. In a preferred embodiment, the peat moss board is a laminated structure comprising a central peat moss core sandwiched between two relatively thin layers of Kraft wood pulp fibers.

As embodied and broadly described herein, the invention further provides an apparatus for applying conditioning agent to fibrous material to impart a desirable property thereto, the apparatus comprising:

- a fluid permeable supporting surface for receiving fibrous material to be treated with the conditioning agent;
- a pumping device in fluid communication with the fluid permeable supporting surface to establish across the fibrous material a pressure differential;
- a conditioning agent applicator station including a foaming device for supplying conditioning agent in a foamed condition to the fibrous material, whereby the pressure differential causes the conditioning agent to penetrate in the fibrous material.

## BRIEF DESCRIPTION OF THE DRAWINGS

- Figure 1 is a schematical view of an apparatus for manufacturing a peat moss board, including a station for applying wetting agent in a foamed condition which is constructed in accordance with the present invention;
- Figure 2 is a perspective view of the station for applying the foam of wetting agent of the apparatus shown in Figure 1;
- Figure 3 is an enlarged perspective view of a station for heating the slurry of peat moss material of the apparatus shown in Figure 1; and
- Figure 4 is a perspective view of an apparatus for conducting a 45° impact capacity test procedure.

## DESCRIPTION OF A PREFERRED EMBODIMENT

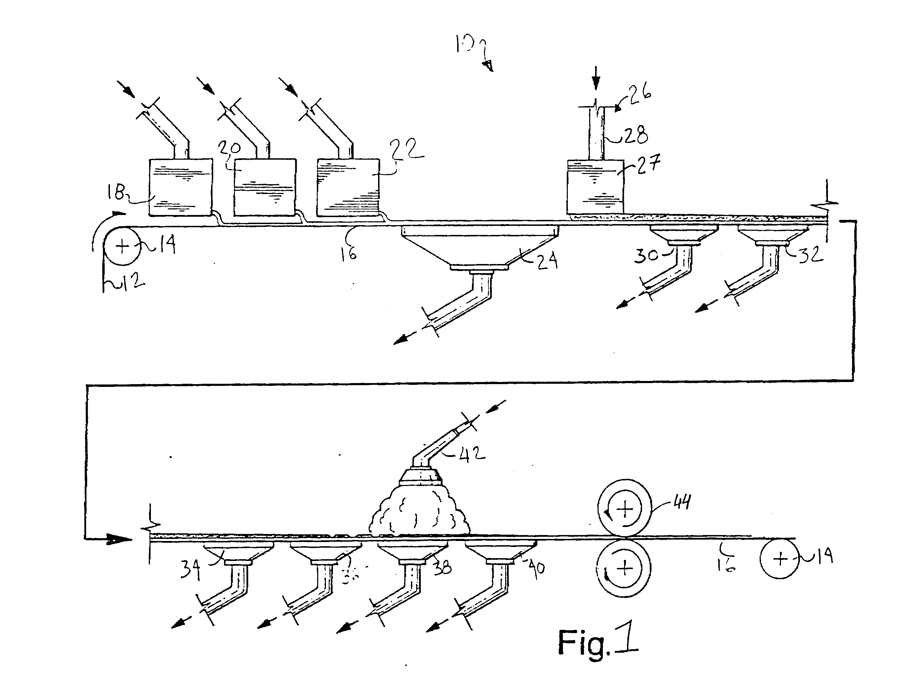

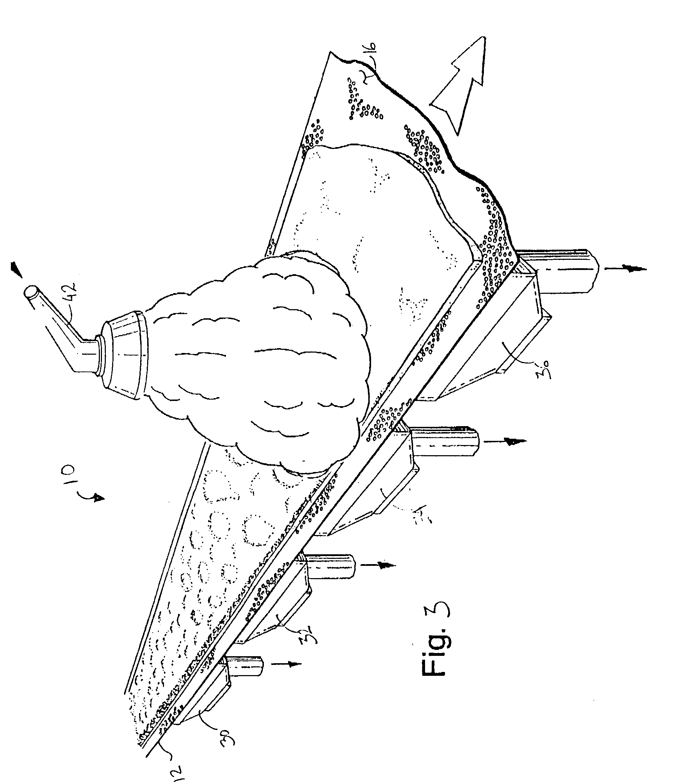

An apparatus for manufacturing a peat moss board is illustrated in Figures 1, 2 and 3. The peat moss board is suitable for use as a primary absorbent component in structures for absorbing body exudate such as sanitary napkins, diapers, adult disposable briefs, urinary pads, tampons, wound dressings and the like.

The apparatus, designated comprehensively by the reference numeral 10, comprises an endless fluid pervious Fourdrinier wire 12 which is mounted on rollers 14 to provide a horizontally extending run 16 which is continuously advanced forward to transport a slurry of peat moss material through various processing stations.

Headboxes 18, 20 and 22 arranged in a spaced apart relationship along the path of travel of the wire 12 are provided to deposit on the wire 12 slurry in sheeted form. The slurry, before being supplied to the headboxes 18, 20 and 22, is passed through a screening station (not shown in the drawings) as it will be described hereinafter. The headbox trio deposits on the wire 12 three layers of slurry in a superposed relationship. Advantageously, this arrangement may be used to form laminated webs by sheeting a slurry of different material from each headbox. Downstream the headboxes 18, 20 and 22 a vacuum slot 24 is provided which is in fluid communication with a vacuum pump (not shown in the drawings) in order to create a low level suction beneath the wire 12 to de-water the slurry of peat moss material.

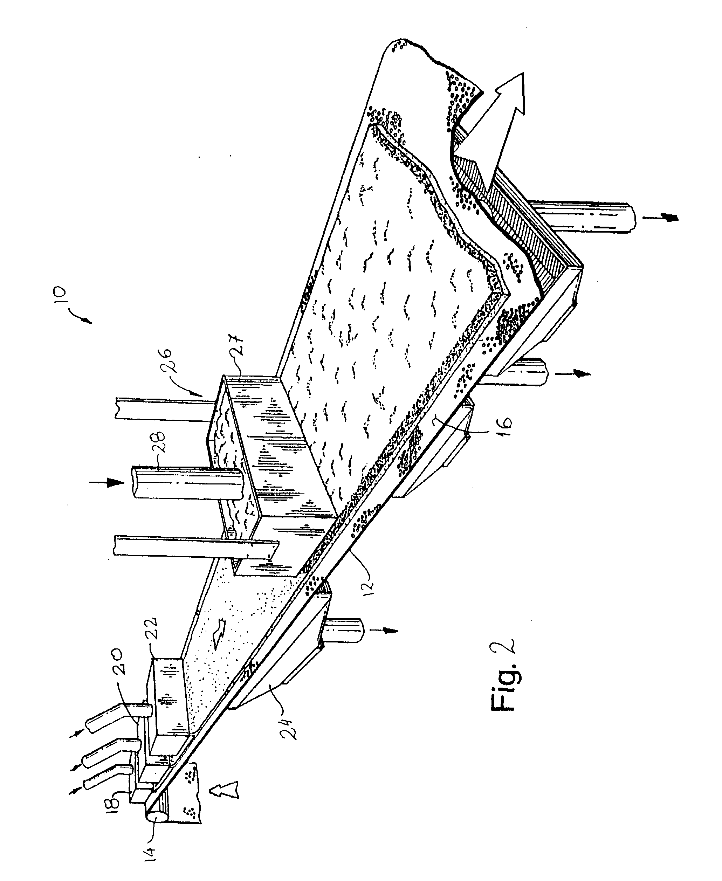

Downstream of the vacuum slot 24 is provided a station 26 to apply on the slurry of peat moss material wetting agent in a foamed condition. The station 26, shown in more detail in Figure 2 comprises an open-bottom, generally rectangular chamber 27 vertically spaced from the top surface of the slurry by a predetermined distance. A foam feed conduit 28 terminates in the open-bottom chamber 27. The foam feed conduit 28 is connected to a foaming unit which mechanically agitates and aerates by injection of compressed air an aqueous solution of surfactant in order to produce the foam that is delivered to the open-bottom chamber 27. The foaming unit is not shown in the drawings nor described in detail in this specification because it is a commercially available piece of equipment. For example, a foaming unit manufactured by EASE INC. of Tunnel Hill, Georgia, U.S.A., has been found satisfactory for practising the invention.

Downstream the open-bottom chamber 27 are arranged six consecutive vacuum slots 30, 32, 34, 36, 38 and 40, that create a relatively high pressure differential across the slurry of peat moss material which increases progressively along the path of travel thereof in order to de-water the slurry and draw the foam of wetting agent in the slurry. The vacuum slots 30 to 40 are connected to respective vacuum pumps (not shown in the drawings).

Near the end of the row formed by the vacuum slots 30 to 40 is provided a steam nozzle 42 in fluid communication with a source of steam under pressure (not shown in the drawings) to direct toward the slurry a relatively low pressure steam jet which has the effect of heating the top surface of the slurry in order to disperse fragments of foam of the wetting agent that have not penetrated in the slurry.

Downstream of the nozzle 42 is provided a press station 44 of a construction well-known to those skilled in the art. The purpose of the press station is to express water from the slurry by a rolling action.

The operation of the apparatus 10 is as follows. The starting peat moss harvested from the bog should have a relatively high absorbent capacity. Peat moss capable of absorbing and retaining at least about 25 and preferably about 50 times its weight in water has been found satisfactory.

The starting peat moss is classified to remove the extremely fine material, commonly referred to as fines, and large pieces of material including roots, branches and the like which do not contribute significantly to the absorbency of the peat moss material.

The classification is carried out such that anything that remains on a number 10 mesh screen (2000 microns) is discarded and anything that passes through a number 200 mesh screen (74 microns) is also discarded. Preferably, anything that remains on a number 14 mesh screen (1410 microns) is discarded and anything that passes through a number 100 mesh screen (149 microns) is discarded.

The classification is carried out by a wet screening process which consist of forming an aqueous slurry of the peat moss material and flowing the slurry through successive screening stages to extract from the slurry the fines and the excessively large particles.

The screened peat moss fraction is then diluted with water to a manageable slurry. If desired, a fibrous component may be added to the slurry. The fibrous component is suitably a natural or synthetic textile fiber such as rayon, polyester, nylon, acrylic or the like, having a length of from about 0.25 to 0.75 inches, preferably about 0.5 inches and a denier of from about 1.0 to 5. The fibrous component may be present in an amount from about 2 to 20% by weight, most preferably from 4 to 8%.

The fibrous component may also include such materials as Kraft wood pulp and mechanical wood pulp. As used herein, the term mechanical wood pulp is meant to include ground wood pulp, thermo-mechanical pulp and refiner wood pulp. Ground wood is essentially trees and branches which have been debarked, cleaned and then ground into particulate matter. Refiner wood pulp differs from ground wood pulp only in that the grinding step utilizes a refiner, i.e. a disc-like device well known in the art and generally having metallic ribs at the peripheral sections thereof which last contact the wood particles and help separate the wood fibers without excessively damaging them. Thermo-mechanical wood pulp is similar to refiner pulp with the exception that the wood particles are heated when in the refiner, usually with steam, and this heating further aids in separating the wood fibers. The common characteristic of these mechanical pulps is that no attempt has been made to separate the fibers by chemical means although they may later, after being reduced to fine particulate matter, be subjected to a desired chemical treatment.

Preferably, when the mechanical wood pulp is used in the peat moss board such mechanical wood pulp has a Canadian Standard Freeness (TAPPI test method T-227) of from about 60 to 750 and preferably from about 400 to 600.

The Kraft wood pulp, also usable in combination with the peat moss is essentially chemically treated, long fibred pulp such as sulfite and sulfate wood pulps.

A slurry of Kraft wood pulp fibers is supplied to the headbox 18 and laid down on the wire 12. The Kraft wood pulp slurry is carried forward by the wire 12 and passes under the headbox 20 to which is supplied the slurry of peat moss material and additives. The peat moss slurry is laid directly on top of the Kraft wood pulp layer. A final layer of Kraft wood pulp slurry is deposited on the peat moss layer by the headbox 22. This structure provides a sandwich-like laminate comprising a central peat moss core covered by two layers of Kraft wood pulp fibers which contribute to enhance the strength characteristics of the final peat moss board.

It is preferred that the Kraft wood pulp fibers employed be bleached and have a Canadian standard freeness of relatively high value, i.e.g. about 450 to 750. While the proportions of each Kraft wood pulp layer to the peat moss layer are not critical, a suitable product results when the layer of about 0.5 to 5 grams of Kraft wood pulp fibers per square foot is employed.

The slurry laminate passes over the vacuum slot 24 which creates a suction of approximately 55 inches of water (inH2O) to de-water the slurry. Immediately after the vacuum slot 24 the slurry has a consistency in the range from about 4 to about 15 % solids. More preferably, the slurry has a consistency of approximately 4.4% solids.

The consistency of the slurry determines its porosity and in turn determines how fast the wetting agent in a foamed condition will penetrate in the fibrous network. Generally speaking, the higher the porosity, the faster the wetting agent will be absorbed therein.

The consistency of the slurry is controlled by varying such factors as the pressure difference during the vacuum de-watering and the speed of the wire 12. Generally, increased vacuum and decreased speed will result into a product having a higher consistency.

A foam of wetting agent is prepared by mechanically agitating in the foaming unit and aerating by the injection of compressed air an aqueous solution of surfactant. A surfactant manufactured by the CLOUGH CHEMICAL COMPANY and commercialized under the name THOROWET G-60 has been found satisfactory. The concentration of surfactant in the aqueous solution is in the range from about 0.20 to about 0.70 % by weight. More preferably, the concentration of surfactant is of approximately 0.38 % by weight.

The foam of wetting agent has a density in the range from about 0.015 to about 0.090 g/cc. More preferably, the density of the foam is of approximately 0.023 g/cc.

The concentration of surfactant in the surfactant solution, the density of the foam and the amount of foam determine the amount of surfactant that is being applied to the slurry. In the example shown, the amount of bone dry surfactant delivered to the slurry weighs in the range from about 1.4 to about 5.8 grams per kilogram of bone dry solids in the slurry. Preferably, the amount of surfactant which is delivered weighs in the bone dry state approximately 2.24 grams per kilogram of bone dry solids in the slurry.

The amount of foam delivered on the slurry is determined by the thickness of the foam layer that leaves the open-bottom chamber 27. This parameter is determined by the vertical spacing of the open-bottom chamber 27 from the top surface of the slurry and can be adjusted as desired to fine tune the amount of surfactant added to the slurry. In the example shown, the thickness of the foam layer is set at approximately 9 millimetres.

The slurry and foam pass over the vacuum slots 30 to 40 which de-water the slurry and draw the foam of wetting agent therein. Advantageously, the suction provided by the vacuum slots increases from one vacuum slot to another along the path of travel of the slurry. In the example shown, the vacuum slots 30 to 40 are operated at suction levels of 6, 7, 9, 14, 14 and 14 inches of mercury (inHg), respectively.

The steam nozzle 42 is supplied with steam at a pressure of approximately 1.4 pounds per square inch (psi) and delivers the steam to heat the top surface of the slurry and to disperse the residual fragments of foam remaining thereon.

When the slurry leaves the last vacuum slot 40 it is passed through the press station 44 for further dewatering.

The method in accordance with the present invention provides a non-uniform dispersion of the wetting agent in the slurry. More particularly, a higher concentration of wetting agent remains on the top surface of the slurry by comparison to the bottom surface thereof. Accordingly, the resulting peat moss board has a preferential fluid absorption surface which is more hydrophilic and has the ability to absorb fluid more rapidly than the other surface of the board.

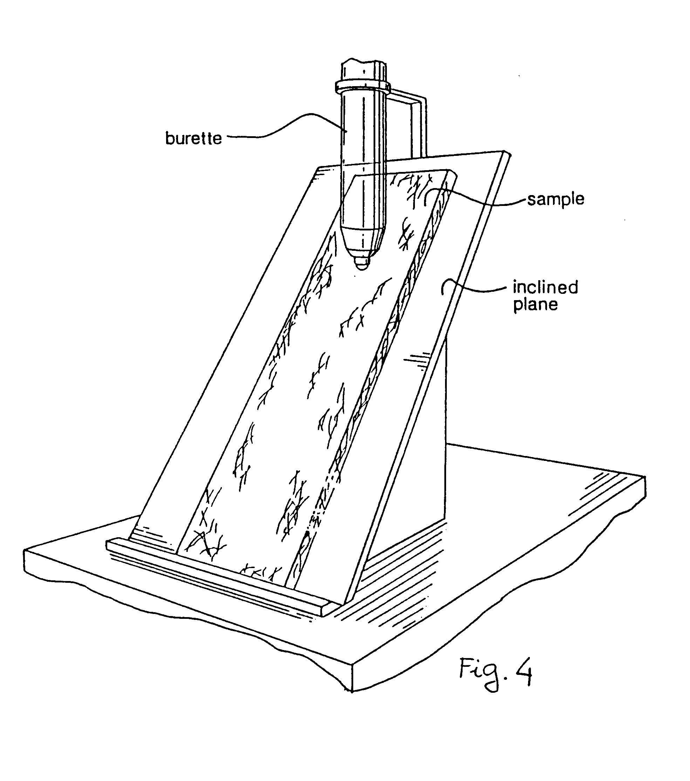

To demonstrate this feature, each surface of the peat moss board is subjected to a 45° impact capacity test. The apparatus for performing the test is illustrated in Figure 4. The purpose of the test is to determine the fluid retention capacity of the peat moss board by measuring its ability to accept and retain a finite discharge of fluid at an inclined plane. The impact capacity on a 4 inch by 7 inch sample is measured by weighing the amount of fluid that is retained in the sample placed on a 45° inclined plane, on which an amount of test fluid which weighs ten times the weight of the sample (stabilized at 12 % moisture) has been released from an overhanging burette. The burette is spaced at 0.5 inch from the sample surface and it is situated at 0.5 inch away from the upper extremity of the sample. The test fluid that is being used is a 1 % saline solution.

The peat moss board subjected to the 45° impact capacity test has a central peat moss core sandwiched between two layers of Kraft wood pulp fibers and has the following formulation:

| INGREDIENTS | PARTS BY WEIGHT* |
|---|---|
| Peat moss | 76.81 |
| Polyester fibers | 4.90 |
| Kraft wood pulp fibers in the peat moss core | 7.85 |
| Kraft wood pulp fibers on the bottom layer | 5.23 |
| Kraft wood pulp fibers on the top layer | 4.58 |

\* Based on the weight of bone dry board.

On the preferential fluid absorption surface, the peat moss board is capable to retain 77.4 % by weight of the test fluid discharge while on the other surface the peat moss retains 62.1 % by weight of test fluid discharge. This test illustrates that the preferential fluid absorbent surface has a 25 % percentage higher fluid absorbency rate than the other surface of the peat moss board.

The scope of the present invention is not limited by the description, examples and suggestive uses herein, as modifications can be made without departing from the spirit of the invention. Applications of the product and the methods of the present invention for sanitary and other health-care uses can be accomplished by any sanitary protection, incontinence, medical and absorbent methods and techniques as are presently or prospectively known to those skilled in the art. Thus, it is intended that the present application covers the modifications and variations of this invention provided that they come within the scope of the appended claims and their equivalents.

**Claims**

1. A method for applying a conditioning agent to a fibrous material to impart a desirable property thereto, said method comprising the steps of:
   - providing a conditioning agent in a foamed condition;
   - depositing said conditioning agent on said fibrous material; and
   - establishing a pressure differential across said fibrous material to draw said conditioning agent therein.

2. A method as defined in claim 1, comprising the step of depositing said conditioning agent on a slurry of said fibrous material, whereby said pressure differential simultaneously extracts dilution fluid from said slurry and draws said conditioning agent in said slurry.

3. A method as defined in claim 2, comprising the step of heating said slurry after said conditioning agent has been deposited thereon for dispersing residual foam fragments of said conditioning agent remaining on said slurry.

4. A method as defined in claim 3, comprising the step of spraying steam toward said slurry for dispersing said residual foam fragments.

5. A method as defined in claim 2, further comprising the steps of:
   - continuously advancing said slurry;
   - providing over said slurry an open-bottom chamber which is stationary with respect to said slurry and spaced apart therefrom by a predetermined distance; and
   - feeding said conditioning agent to said open-bottom chamber, whereby said conditioning agent is deposited over said slurry and egresses said open-bottom chamber as a foam layer having a thickness corresponding approximately to said predetermined distance.

**6.** A method as defined in claim 1, wherein said conditioning agent is a wetting agent, said method comprising the steps of adding surfactant to a dilution agent to form a surfactant solution and agitating said surfactant solution to foam said solution in order to provide said wetting agent.

**7.** A method as defined in claim 6, further comprising the step of aerating said surfactant solution.

**8.** A method for applying wetting agent to peat moss material, said method comprising the steps of:
- forming an aqueous slurry of peat moss material;
- sheeting said slurry;
- providing wetting agent in a foamed condition;
- depositing said wetting agent on said slurry; and
- establishing a pressure differential across said slurry to de-water said slurry and to draw said wetting agent in said slurry.

**9.** A method as defined in claim 8, further comprising the steps of:
- continuously advancing said slurry;
- providing over said slurry an open-bottom chamber (27) which is stationary with respect to said slurry and spaced apart therefrom by a predetermined distance; and
- feeding said wetting agent to said open-bottom chamber, whereby said wetting agent is deposited over said slurry and egresses said open-bottom chamber (27) as a foam layer having a thickness corresponding approximately to said predetermined distance.

**10.** An apparatus for applying conditioning agent to fibrous material to impart a desirable property thereto, said apparatus comprising:
- a fluid permeable supporting surface (16) for receiving fibrous material to be treated with said conditioning agent;
- a pumping device in fluid communication with said fluid permeable supporting surface (16) to establish across said fibrous material a pressure differential;
- a conditioning agent applicator station (26) including a foaming device for supplying conditioning agent in a foamed condition to said fibrous material, whereby said pressure differential causes said conditioning agent to penetrate in said fibrous material.

Fig. 1

EP 0 546 587 A1

Fig. 2

Fig. 3

burette

sample

inclined
plane

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-2 318 269 (CHEMISCHE INDUSTRIE AKU-GOODRICH B.V.) | 1-3,5,10 | A61F13/00 D21H21/56 D21H23/46 |
| A | * page 2, line 17 - line 39 *<br>* page 4, line 14 - line 15 *<br>* page 4, line 38 - page 5, line 3 *<br>* figures * | 6-9 | |
| X | US-A-4 385 954 (PAULS ET AL)<br>* column 3, line 47 - line 57 *<br>* column 4, line 28 *<br>* column 5, line 13 - line 21 *<br>* figure 1 * | 1-3,10 | |
| X | DE-A-2 164 902 (FA. CARL FREUDENBERG)<br>* page 2, last paragraph *<br>* page 5, paragraph 2 *<br>* claims 1,2 * | 1,2,10 | |
| A | US-A-4 099 913 (WALTER ET AL)<br>* column 2, last paragraph * | 1,8 | |
| A | GB-A-1 246 910 (JOHNSON & JOHNSON)<br>* page 3, line 34 - line 45; figures 1,5 * | 1 | TECHNICAL FIELDS SEARCHED (Int. Cl.5 )<br><br>A61F D21H |
| A | GB-A-1 071 191 (JOHNSON & JOHNSON)<br>* page 7, line 84 - line 118 *<br>* figures 23-25 * | 1 | |

-----

The present search report has been drawn up for all claims

| Place of search | Date of completion of the search | Examiner |
|---|---|---|
| THE HAGUE | 19 FEBRUARY 1993 | SCHöLVINCK T.S. |

EPO FORM 1503 03.82 (P0401)